(19) Europäisches Patentamt
European Patent Office
Office européen des brevets

(11) Publication number : **0 447 491 B1**

# (12) EUROPEAN PATENT SPECIFICATION

(45) Date of publication of patent specification : **11.08.93 Bulletin 93/32**

(21) Application number : **90901350.0**

(22) Date of filing : **08.12.89**

(86) International application number : **PCT/US89/05599**

(87) International publication number : **WO 90/06748 28.06.90 Gazette 90/15**

(51) Int. Cl.[5] : **A61K 31/24,** A61K 31/41, A61K 31/44, A61K 31/50, A61K 31/535

## (54) Protoporphyrinogen inhibitors and their use in compositions for detecting and treating Tumours.

(30) Priority : **12.12.88 US 285151**
**07.04.89 US 335007**
**03.05.89 US 351331**

(43) Date of publication of application : **25.09.91 Bulletin 91/39**

(45) Publication of the grant of the patent : **11.08.93 Bulletin 93/32**

(84) Designated Contracting States : **BE ES FR IT NL**

(56) References cited :
**US-A- 4 393 071**
**Pesticide Biochemistry and Physiology, vol. 32, 1988, Academic Press, Inc. (New York, US); M. Matringe et al., pp. 164-172**
**Plant Physiol., vol. 87, 1988 (Bethesda, US); D.A. Witkowsky et al., pp. 632-637**
**Pesticide Biochemistry and Physiology, vol. 31, 1988, Academic Press, Inc. (New York, US); J. Lydon et al., pp. 74-83**
**J. Pesticide Sci, vol. 11, 1986 (Tokyo, JP) ; K. Wakabayashi et al., pp. 635-640**
**The New England Journal of Medicine, vol. 302, no. 14, 3 April 1980 (Boston, US); D.A. Brenner et al., pp. 765-768**
**Human Genetics, vol. 58, 1981, Springer Verlag (Berlin, DE); J. Deybach et al., pp. 425-428**
**Biochemical and Biophysical Research Communications, vol. 106, no. 3, 15 June 1982, Academic Press, Inc. (New York, US); J.M. Camadro et al., pp. 724-730**
**FEBS Letters, vol. 245, no. 1,2, 1989 Elsevier (Amsterdam, NL); M. Matringe et al., pp. 35-38**
**Br. J. Cancer, vol. 56, no. 5, 1987 (London, GB); Z. Malik et al., pp. 589-595**

(56) References cited :
**Zeitschrift für Naturforschung, vol. 43, no. 9-10, 1988, Verlag der Zeitschrift für Naturforschung (Tübingen, DE); G. Sandmann et al., pp. 699-704**
**Mol. Gen. Genet., vol. 188, no. 1, 1982 Springer Verlag (Berlin, DE); M.S. Crawford et al., pp. 1-6**

(73) Proprietor : **FMC CORPORATION**
**1735 Market Street**
**Philadelphia, PA 19103 (US)**

(72) Inventor : **HALLING, Blaik, Phillip**
**1556 Willow Pond Drive**
**Yardley, PA 19067 (US)**
Inventor : **WITKOWSKI, Debra, Ann**
**19 Cori Street**
**Parlin, NJ 08859 (US)**

(74) Representative : **Rodhain, Claude et al**
**Cabinet Claude Rodhain 30, rue la Boétie**
**F-75008 Paris (FR)**

EP 0 447 491 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the photodynamic treatment of mammalian tumor cells, as in the photodynamic treatment of cancer.

It is by now well known to supply hematoporphyrin derivative or a mixture of porphyrins derived therefrom (e.g. Photofrin II®) to mammalian tumor cells and then subject such cells to light of appropriate wavelength to effect cell destruction. Such photodynamic therapy is the subject of a series of articles making up a special issue of Photochemistry and Photobiology, Volume 46, number 5, November, 1987 (hereinafter "P&P 46-5"). As shown in those articles, such photodynamic therapy has been used in the treatment of a wide variety of cancers (including cancers of the bronchial tubes, bladder, esophagus, lung, skin, head and neck, brain, and colon and intraocular and gynecologic cancers). It has been stated that the biochemical effects of such porphyrin photosensitization include the cross-linking of membrane proteins, the peroxidation of lipids, the inhibition of transport of some essential metabolites, the lysis of lysosomal and mitochondrial membranes, the inactivation of several enzymes and an increased cell uptake of porphyrins (P&P 46-5, page 695). The porphyrin material is generally injected (e.g. intravenously or intra-peritoneally), a typical dose being about 2 mg of Photofrin II per kg of body weight.

The porphyrin material may also be incorporated into a liposome and injected (e.g. intraperitoneally), as disclosed for instance in Spikes et al., "Photodynamic Behavior of Porphyrins in Model Cell, Tissue and Tumor Systems"; in Photodynamic Therapy of Tumors and Other Diseases (Edited by G. Jori and C.A. Perria); pages 45-53; Libreria Progetto, Padua (1985), and references cited therein.

The technical literature indicates that the introduction of protoporphyrin into cells, such as human erythrocytes (Dubbelman et al, Biochimica et Biophysica Acta, 511 (1978) 141-151) or murine leukemia cells (Kessel, Cancer Research 42, 1703-1706 May 1982), has a marked photosensitizing effect. The Kessel article states that it was the most potent photosensitizing agent of those tested. However, when Kessel and others (e.g. Berenbaum et al, Br. J. Cancer (1982) 45, 571-581) injected protoporphyrin into animals they found no detectable protoporphyrin in the tumors, indicating that protoporphyrin introduced as such into the body may be readily lost into the circulation.

It is also well known to employ hematoporphyrin derivative and similar materials for the detection and localization of tumors, such as cancer of the bladder or lungs (P&P 46-5, page 759, for instance).

In this invention the agent for treating the cells in such photodynamic treatment, or in the known methods for detection and localization of tumors, is not a porphyrin, or is not solely a porphyrin. Instead it comprises an enzyme-inhibitor agent which inhibits the enzymatic conversion of protoporphyrinogen to heme in said cells, thereby causing a buildup of endogeneous protoporphyrin IX in said cells. We have ascertained that agents, such as certain types of herbicidal compounds, which inhibit the enzymatic conversion of protoporphyrinogen to chlorophyll in plant cells also inhibit the enzymatic conversion of protoporphyrinogen to heme in mammalian cells. We believe that the inhibition by such agents probably affects the step of conversion of protoporphyrinogen to protoporphyrin IX by an enzyme (protoporphyrinogen oxidase, EC 1.3.3.4), so that the protoporphyrinogen cannot follow the normal enzymatic pathway to protoporphyrin IX but instead becomes oxidized in the cell (e.g. in the plasma) but outside the normal enzymatic pathway (e.g. outside the organelle membrane), and that the result is an accumulation of protoporphyrin IX in locations where it is unavailable for the normal conversion to heme, with the result that, when the cells are subjected to light, this accumulated protoporphyrin IX has a cell-destroying effect like that exhibited in the above-described photodynamic treatment.

The enzyme inhibiting agents of this invention are specific inhibitors of protoporphyrinogen oxidase in the sense that they do not operate as general enzyme poisons such as denaturing or cross-linking agents (e. g. sulfhydryl reagents), preferably they are not materials which affect the oxidation conditions such as electron acceptors; thus, preferred agents for this invention have redox potentials more negative than about -500 mV, such as more negative than -800 mV (measured, for instance in conventional manner in an aprotic solvent for the agent, as by cyclic voltametry or polarographically). It is also preferred that the agent not be a tetrapyrrole and that its $I_{50}$ for protoporphyrinogen oxidase be less than about 1 $\mu$M such as less than about 0.3 $\mu$M, e. g. an $I_{50}$ of about 0.1 or 0.03 or 0.01 $\mu$M or less.

The products encompassed by the present invention are recited in independent claims 1 and 2 (under the form of a pharmaceutical composition , or 3 to 7 (inhibitor as such).

The enzyme-inhibiting agent is preferably one which has a high capacity for disrupting the plasmalemma of plant material. One test for that capacity is the Efflux Experiment described in the article by Halling and Peters in Plant Physiology, 84, 1114-5 (1987). In such a test (described in more detail in Appendix A below), preferred agents show a total efflux of at least 50% at a treatment rate of 100 $\mu$M, preferably at a treatment rate of 1 $\mu$M or less, such as 100 nM; highly active materials, such as 1-(4-chloro-2-fluoro-5-propargyloxyphenyl)-3-methyl-4-difluoromethyl-$\Delta^2$-1,2,4-triazolin-5-one or lactophen (described below), give

total efflux percentages of over 90% at 100 nM concentration.

Another test of the capacity of a material for disrupting the plasmalemma of plant material is the Light-Induced Greening Inhibition Test described more particularly in Appendix B below. This test measures the capacity to inhibit the light-greening of dark-bleached Chlamydomonas reinhardi mutant y-l (a type of algae which when grown in the dark does not make chlorophyll, so that the mass of algae becomes bleached owing to the presence of new non-green cells, and which produces chlorophyll again when it is exposed to light). Many of the preferred compounds (agents) used in this invention have the ability to inhibit the light-greening by at least 50% when the compound is used at a concentration of $10^{-5}$M, more preferably at a concentration of $10^{-6}$M or less, e.g. $10^{-7}$M. In addition, the compound should be one which, when used at said concentration in the Light-Greening Inhibition Test, gives a supernatant which shows a light absorption peak at about 405 nm which is higher than the chlorophyll peak (the peak at about 668 nm in this system), e.g. the supernatant shows a 405 nm peak whose height is 2, 3 or 4 times the height of the 668 nm peak.

Among the enzyme-inhibiting agents which may be employed in the practice of this invention are herbicidal compounds of the following classes (A to D):

A. Aryl heterocyclic herbicides of the general formula

Ph - NHet

where "Ph" is a substituted phenyl, preferably 2,4-di-substituted phenyl, more preferably a 2,4,5-trisubstituted phenyl, and NHet is a 5- or 6-membered heterocyclic ring having one to four ring-nitrogen atoms and having the formula

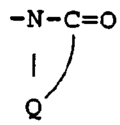 or 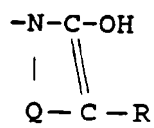 or 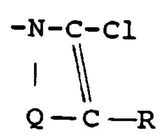

where Q represents the balance of the heterocyclic ring and R represents hydrogen or a substituent group. This class of compounds includes such materials as those designated as "the cyclic imide class of herbicides" in an article by Wakabayashi et al., J. Pesticide Sci. 11, 635-460 (1986) which shows the following compounds in its Fig. 1:

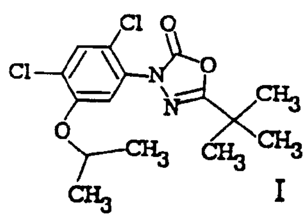
I

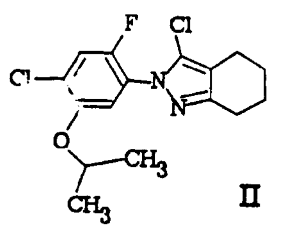
II

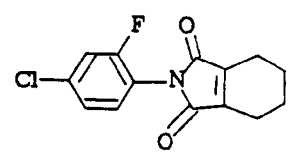
III

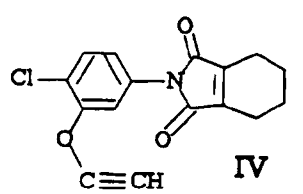
IV

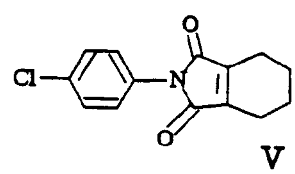
V

Compound I is an aryl oxadiazole herbicide, namely 2-tert-butyl-4-(2,4-dichloro-5-isopropoxyphenyl)-$\Delta^2$-1,3,4-oxadiazolin-5-one. Other 2-alkyl-4-(2,4,5-tri-substituted phenyl)-$\Delta^2$-1,3,4-oxadiazolin-5-ones which may be used in the present invention are disclosed, for instance, in US-A-3 385 862; US-A-3 836 539; and US-A-3 876 413.

Compound II is an aryl tetrahydroindazole herbicide, namely 3-chloro-2-(4-chloro-2-fluoro-5-isopropoxyphenyl)-4,5,6,7-tetrahydro-2H-indazole. Other 3-substituted-2-(2,4,5-trisubstituted phenyl)tetrahydro indazoles which may be used in the present invention are disclosed, for instance, in US-A-4 670 043.

Compounds III, IV and V are aryl tetrahydrophthalimide herbicides. Other aryl tetrahydrophthalimides which may be employed in the present invention are disclosed, for instance, in US-A-4 431 822; US-A-4 670 046; US-A-4 670 042; and US-A-4 439 229 and WO-A-87/07602. The latter two citations above also disclose other NHet rings which may be used, such rings being illustrated, for example, at column 4 line

25 to column 5 line 20 of US-A-4 439 229 and at pages 12 to 14 of WO-A-87/07602.

Other suitable herbicides of the PH-NHet type are:

aryl triazolinones, such as those disclosed in US-A-4 318 731; US-A-4 398 943; US-A-4 404 019; US-A-4 702 945; US-A-4 705 557; US-A-4 702 763; US-A-4 761 174 and WO-A-85/01637, WO-A-85/04307, WO-A-87/00730, WO-A-87/03782, WO-A-86/04481 and WO-A-88/01133;

ary tetrazolinones, such as those disclosed in US-A-4 734 124, WO-A-85/01939 and WO-A-87/03873;

aryl triazinediones such as those disclosed in US-A-4 755 217, US-A-4 766,233 and WO-A-86/00072;

aryl hydantoins, such as those disclosed in US-A-4 427 438;

aryl urazoles, such as those disclosed in US-A-4 452 981; and

aryl hexahydropyridazines, such as those disclosed in US-A-4 619 687.

B. Aryl heterocyclic urethanes, such as those disclosed in US-A-4 521 242.

C. Phenyl ether herbicides, such as those having a p-halo or p-nitro phenoxyphenyl structure, such as the following commercially available materials:

5-(2-chloro-4-(trifluoromethyl) phenoxy)-2-nitro-N-methanesulfonylbenzamide (fomesafen)

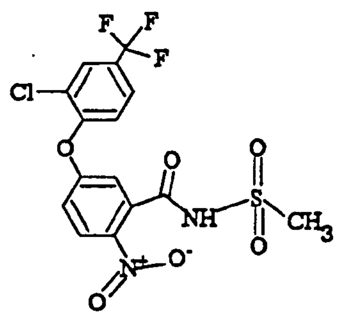

methyl 5-(2,4-dichlorophenoxy)-2-nitrobenzoate (bifenox)

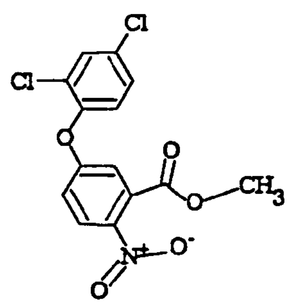

sodium 5-(2-chloro-4-(trifluoromethyl)phenoxy)-2-nitrobenzoate (acifluorfen)

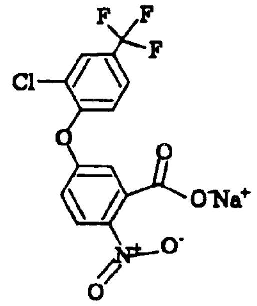

2-chloro-1-(3-ethoxy-4-nitrophenoxy)-4-trifluoromethylbenzene (oxyfluorfen)

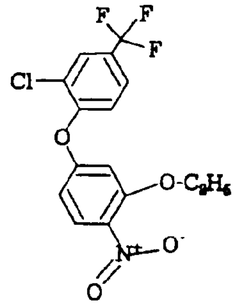

Other suitable commercially available diphenyl ether herbicides are

lactophen: 1-(carboethoxy)ethyl 5-(2-chloro-4-trifluoromethylphenoxy)-2-nitrobenzoate,
fluoroglycofen: (carboethoxy)methyl 5-(2-chloro-4-trifluoromethylphenoxy)-2-nitrobenzoate,
fromesofen: 5-(2-chloro-4-(trifluoromethyl)phenoxy)-N-methylsulfonyl-2-nitrobenzamide,
chloronitrofen: 2,4,6-trichloro-(4-nitrophenoxy)-benzene,
fluorodifen: 2-nitro-1-(4-nitrophenoxy)-4-trifluoromethylbenzene,
nitrofen: 2,4-dichloror-1-(4-nitrophenoxy)benzene,
chlomethoxyfen: 4-(2,4-dichlorophenoxy)-2-methoxy-1-nitrobenzen.

Still another suitable diphenyl ether herbicide is methyl 5-(2-chloro-4-trifluoromethylphenoxy)-2-nitroacetophenone oxime-O-acetate.

D. Aryl pyrazole herbicides such as those disclosed in US-A-4 563 210; US-A-4 496 390; US-A-4 459 150 and DE-A-35 20 327.

E. Compounds of the formula

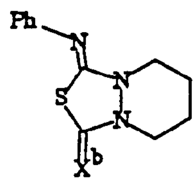

or

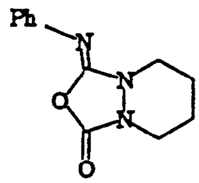

where $X^b$ is O or S and "Ph" has the meaning described above, such as compounds disclosed in EP-A-273 417 or Derwent Abstracts accession no. 87-040749.

Treatment with the agents of this invention may be effected by intravenous or intraperitoneal injection. The agent may be used as a sterile composition comprising the agent dissolved or dispersed in a pharmaceutically acceptable carrier, for example as an aqueous isotonic solution such as aqueous saline (e.g. 0.9% NaCl) or Dulbecco's phosphate buffered saline (PBS) at a concentration of, say, 2.5 mg $mL^{-1}$, or comprising the agent in a liposomal system such as one prepared with a phospholipid vesicle in a manner such as described at pages 1659-1660 of Remington's Pharmaceutical Sciences, 1985, (17th edition), pusblished by Mack Publishing Company. For instance, analogously to the formulation described by Jori et al., Br. J. Cancer (1983), 48 at page 307, 51.4 mg. of dipalmitoyl-diphosphatidyl-choline may be dissolved in 10 mL of a 1 mM solution of the agent in chloroform-methanol (9:1, v/v), and after thorough mixing the solvent may be removed under vacuum at 30°C, giving a solid which may be resuspended in 10 mL of 0.01M phosphate buffer at pH 7.4 containing 150 mM NaCl, and the cloudy solution then sonicated for 30 minutes at 50°C.

The enzyme-inhibiting agents which may be employed in the practice of this invention may be conjugated or linked to appropriate tumor-specific monoclonal anti-bodies (MABs), using linker technology known in the art, as a means for targeting the enzyme-inhibiting agent to particular tumor sites.

The enzyme-inhibiting agents used in this invention may also be used by simple oral ingestion, preferably in diluted form together with a pharmaceutically acceptable carrier, as by adding them to food as illustrated in Example 4 below.

It may be desirable, especially for oral administration, to employ enzyme-inhibiting agents which are water-soluble salts or which are acidic and form water-soluble sodium or potassium salts, such as an acidic sulfonamide of the type disclosed in WO-A-87/03782 (e.g. agent 6c in Example 4 below) or WO-A-85/001939 and WO-A-87/037873 or a water-soluble salt thereof, or a carboxylic acid or water-soluble salt thereof such as the sodium salt known as acifluorfen.

The enzyme-inhibiting agents which may be used in this invention may be incorporated into conventional pharmaceutical preparations such as tablets (e.g. compressed tablets which may be coated, as with sugar paste and/or syrup), suppositories, capsules (e.g. hard gelatine capsules), suspensions, solutions, powders or ampules. In such preparations the agent may be present in admixture with a pharmacologically acceptable solid and/or liquid carrier which may, if desired, be a nutrient; for example it may be a solid such as corn starch or a liquid diluent, such as water or an edible oil or mineral oil or a solvent e.g. dimethyl sulfoxide. Mixtures of the enzyme-inhibiting agents may be used, e.g. a mixture of agent 6c of Example 4 below and acifluorfen in, for instance, approximately equal proportions. The dosage to be employed may be determined by routine experimentation, well known in the art, such as experiments of the type described, for Photofrin II, in the article by Dougherty on "Photodynamic Therapy (PDT) of Malignant Tumors" in CRC Critical Reviews in Oncology/Hematology vol. 2 issue 2 (1984), pages 83-116.

The following Examples are given to illustrate this invention further.

EXAMPLE 1

HeLa cells (a human tumor cell line commonly employed for tumor research and obtained from the American Type Culture Collection), in logarithmic growth phase (having been cultured at 37°C for five days in one-liter Falcon tissue culture flasks), were washed in phosphate buffered saline (PBS). A 0.25% solution of trypsin in PBS (2 mL) was added, and, after several minutes, the cells were gently removed from the flask and placed into 110 mL of a 25 mM solution of Hepes in Minimum Essential Medium (MEM) supplemented with 10% v/v inactivated calf serum, 1.1 mL of a 200 mM solution of glutamine in 0.85% saline solution, and 11,000 units of penicillin/11,000 mcg streptomycin.

Then, 5.0 mL aliquots of the resulting resuspended cell culture were each placed into a 50 mL Falcon tissue culture flask and (except for the control) mixed with the treating agent. The aliquots were then incubated in the dark at 37°C for three days. The cells were then removed and extracted under green light in the following manner:

Cells were removed from the bottom of the flask with the addition of 0.8 mL of a 0.25% solution of trypsin in PBS. After 1 hour of incubation in the dark, cells and medium were removed from the flasks, placed into round bottom centrifuge tubes, and then subjected to two rounds of freeze-thaw conditions to disrupt the cells and allow for extraction.

Examination of cell suspensions after this disruption regime revealed no intact cells. Ten mL of basic acetone (90% acetone:10% of 0.1 N $NH_4OH$) was then added to each tube, and the tubes were centrifuged to remove protein and cell debris. Five mL of water was added to the supernatant, followed by 0.5 mL of a saturated aqueous solution of NaCl. The pH was then adjusted to 6.8 by the dropwise addition of 2 M $KH_2PO_4$. The aqueous acetone extract was then loaded onto a C8 Baker Prep column. The columns were dried and then washed with 2 mL of water. The tetrapyrroles were eluted with 2 x 1.5 mL volumes of 90/10 $CH_3OH/H_2O$. Extracts were then quantified on a spectrofluorometer.

The treating agents used in this experiment were:

A. 5-amino levulinic acid, which is a known precursor of tetrapyrroles.

B. Acifluorfen-methyl having the formula:

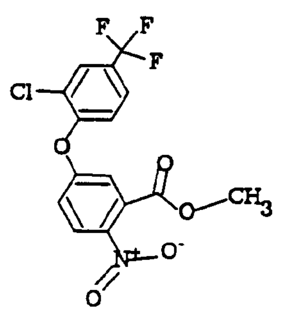

C. A herbicide of the formula:

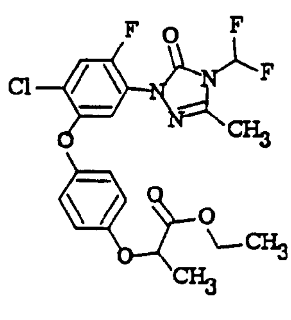

D. A herbicide of the formula:

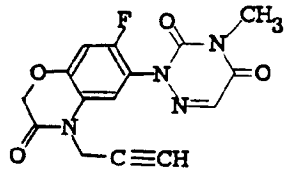

E. A herbicide of the formula:

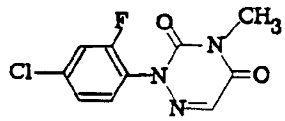

Agent A was supplied as a filter-sterilized 250 mM solution in water, pH 6.5. Agents B, C, D, and E were supplied as 50 mM solutions in acetone. Acetone was also added to the control to provide a concentration of 0.2% v/v therein. The amounts of the agents added to the cell cultures were such as to give the concentrations specified in the Table 1 below. The amounts of the tetrapyrrole protoporphyrin IX ("Proto IX") produced are shown in that Table.

TABLE 1

Tetrapyrrole accumulation in treated HeLa cells

| Agent | Concentration of Agent [µM] | Fluorescence Emission cps 400/630 | Amount of Proto IX (pmoles) |
|---|---|---|---|
| A | 5000 | 41842 | 4.2 |
| B | 100 | 12921 | 1.3 |
| C | 100 | 33477 | 3.5 |
| D | 100 | 10551 | 1.1 |
| E | 100 | 8967 | 0.9 |
| control | | 2795 | 0.3 |

EXAMPLE 2

HeLa cells, in logarithmic growth phase having been cultured for 6 days in six one-liter Falcon tissue culture flasks at 37°C), were washed in phosphate buffered saline (PBS). A 0.25% solution of trypsin in PBS was added, and, after several minutes, the cells were gently removed and placed into 110 mL of a 25 mM solution of Hepes in Minimum Essential Medium (MEM), supplemented with 10% v/v inactivated calf serum, 1.1 mL of a 200 mM solution of glutamine in 0.85% saline solution, and 11,000 units of penicillin/11,000 mcg streptomycin.

Aliquots (5.0 mL each) of the resuspended cell culture were placed into 50 mL Falcon tissue culture flasks, with or without herbicide, and incubated in the dark at 37°C for 4 days, the herbicide being added in dilute acetone solution as in Example 1. Acetone was also added to the controls to provide a concentration of acetone of 0.2% v/v therein. The amount of herbicide was such as to provide the concentrations specified in Table 2 below.

The treating agents were:

B. As in Example 1

C. As in Example 1

F. A herbicide of the formula:

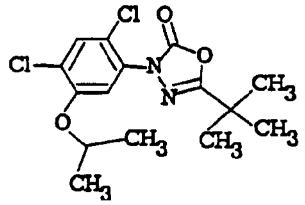

Extraction: The media from each of the flasks were placed in glass round bottom tubes, while the cells adhering to the bottom of the flasks were loosened by the addition of 0.5 mL of a 0.25% solution of trypsin in PBS, and after several minutes at 37°C, then washed from the flasks and recombined with their media. Aliquots of 100 μL were then removed from each cell suspension to determine cell density. The remaining 5.4 mL of cell suspension was then sonified for 30 minutes to disrupt the cells.

Ten mL of basic acetone (90% acetone:10% 0.1 N $NH_4OH$) was then added to each tube, and the tubes were centrifuged to remove protein and cell debris. Five mL of water was added to the supernatant, followed by 0.5 mL of a saturated aqueous solution of NaCl. The pH was then adjusted to 6.8 by the dropwise addition of 2 M $KH_2PO_4$. The aqueous acetone extract was then loaded onto a C8 Baker Prep column. The columns were dried and then washed with 2 mL of water. The tetrapyrroles were eluted with 3 mL of 90/10 $CH_3OH/H_2O$. As in Example 1, all these extraction procedures were carried out substantially entirely under non-protoporphrin IX-exciting light (e.g. green light) or in the dark (e.g. in opaque covered containers). The fluorescence of the extracts was then determined on a SPEX spectrofluorometer. Protoporphyrin IX ("Proto IX") accumulation was quantified utilizing predetermined extinction coefficients. Results are given in Table 2 below.

TABLE 2

Mean Growth Inhibition and Proto IX Accumulation

| Agent | Concentration of Agent (μM) | % Growth Inhibition | Amount of Proto IX (pmoles)** | Relative % Proto IX*** |
|---|---|---|---|---|
| B | 100 | 99 | 5.20 | 2364 |
| F | 100 | 106 | 2.71 | 1232 |
| C | 100 | 52 | 4.41 | 2005 |
| C | 10 | -15* | 0.57 | 259 |
| C | 1 | -26* | 0.19 | 86 |

*Negative values for "Growth Inhibition" indicate that growth occurred.

**Average amount per $10^6$ cells.

***Percent of control.

EXAMPLE 3

Determination of $I_{50}$

Protoporphyrinogen IX ("Protogen IX"). Proto IX was purchased from Porphyrin Products, Logan, UT and purified as outlined by T. P. Fuesler et al., Plant Physiol., 67, 246-249 (1981). Protogen IX was freshly prepared by reduction of Proto IX with a Na/Hg amalgam as outlined by N. J. Jacobs and J. M. Jacobs, Enzyme, 28, 206-219 (1982), utilizing the purified Proto IX at a concentration of 300 μM.

Plant Material. Cucumber (Cucumis sativus L. cultivar 'Wisconsin SMR18') was raised in a dark growth chamber on vermiculite irrigated with a commercial (9-45-15) fertilizer. The seedlings were grown at 25° C and a relative humidity of 80 to 90%. Intermittent illumination, one minute of light per 60 minute cycle with a measured intensity of 25 μE/m$^{-2}$ sec$^{-1}$ (PAR), was supplied by a General Electric 'Bright Stick' controlled by an electronic timer. This provided tissue capable of rapid chlorophyll synthesis while minimizing starch reserves and initial chlorophyll levels.

Chloroplast Isolation. Developing chloroplasts were isolated as described by T. P. Fuesler et al., Plant Physiol., 75, 662-664 (1984), except that in the final purification step, plastids were centrifuged through a 40% rather than 45% (v/v) Percoll cushion. The chloroplasts were resuspended in an assay buffer containing 0.5 M mannitol, 20 mM TES, 10 mM HEPES, pH 7.7, 1 mM EDTA, 1 mM $MgCl_2$, 1% (w/v) bovine serum albumin and 1 mM dithioerythritol to a final concentration of 2 mg protein/mL.

Assays of Protoporphyrinogen Oxidase. Assays were conducted as outlined by J. M. Jacobs and N. J. Jacobs, Arch. Biochem. Biophys., 229, 312-319 (1984). Samples (0.2 mL) of the chloroplasts suspension were preincubated in the dark for 15 minutes with various concentrations of acifluorfen-methyl ("AFM") or with 0.2% (v/v) acetone (as a "control"). Then fifty μL of freshly prepared Protogen (about 15 nM), was added to the suspension to initiate the reaction. Assays were terminated by the addition of 2.75 mL of the fluorometric media of N. J. Jacobs and J. M. Jacobs, Enzyme, 28, 206-219 (1982) consisting of 1% (v/v) Tween-20 (polyoxyethylene sorbitan monolaurate), 50 mM Tris-HCl, pH 8.5, 1 mM EDTA; 1 mM dithioerythritol was substituted for 5 mM gluthathione. The suspensions were then read directly on a SPEX Fluorolog-2 spectroflourometer equipped with a frontface fluorescence option for turbid biological samples. The amount of Proto IX produced was quantified from a standard curve (of quantity of Proto IX versus emmission at 630 nm, on excitation at 400 nm) generated from purified Proto IX in fluorometric media.

To determine the amount of nonenzymatic oxidation to Proto IX in the above assay, the same assay was carried out except that in place of the suspension of active chloroplasts, there was used a suspension in which the chloroplasts had been inactivated by heating for 15 minutes at 85°C. Substracting the quantity of Proto IX thus produced from the quantity produced in the presence of the active chloroplasts gave the amount of Proto IX formed enzymatically. The results are shown graphically in Figure I (in which LSD indicates least significant difference, as is conventional). Figure I shows that the $I_{50}$ value (the concentration which provides 50% inhibition) is less than 0.1 μM, i. e., about 0.03 μM.

Assays of the effect of 10 μm AFM on the enzymatic conversion of Proto IX to magnesium Proto IX in the chloroplast suspension (in the presence of ATP) showed that AFM had no inhibiting effect on that conversion.

EXAMPLE 4

In this Example the enzyme-inhibiting agents were added to the feed of tumor-bearing mice; for some of the mice, used as a control, no agent was added to the feed. The mice were then sacrificed and their kidneys, intestines, adrenals, liver and tumor were removed and analyzed for their content of Proto IX. The following enzyme-inhibiting agents were employed:

6a.

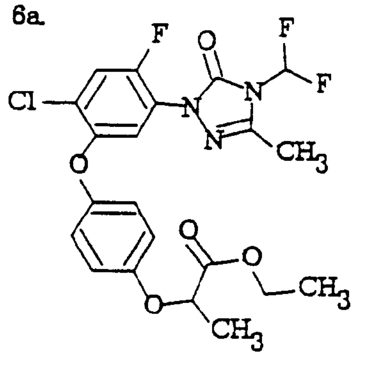

6b.

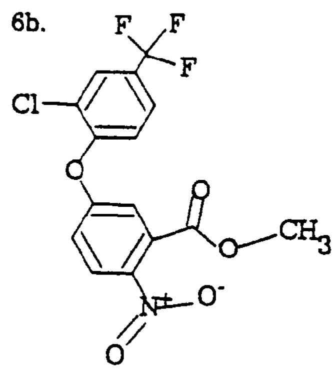

6c.

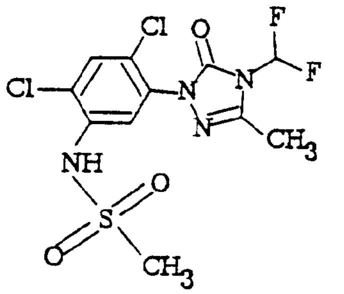

6d.

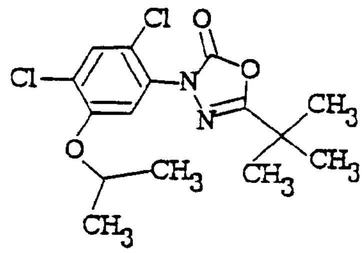

6e.

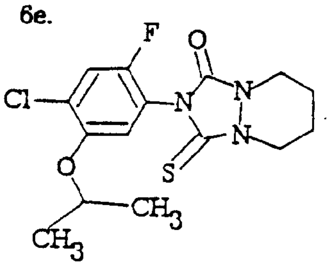

6f.

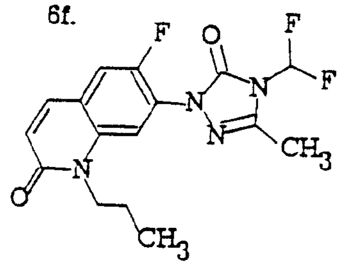

6g.

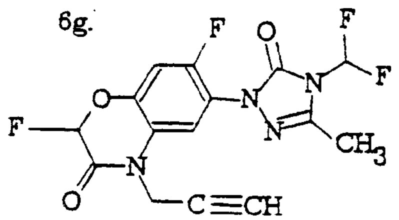

6h.

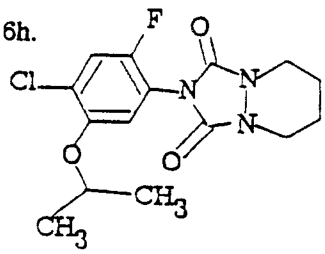

6i.

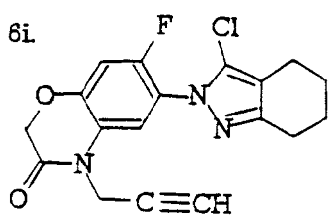

6j.

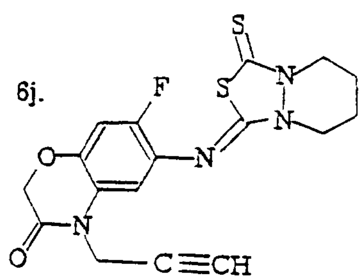

More particularly, the mice were DBA/2 Ha mice which had been injected with SMT-F subcutaneous tumors on Day 0 in the right shoulder. The mice, individually housed, received untreated Purina Rodent Chow 5001 Mash on Day 1, thereafter on Days 2 through 10 the mice received the same Mash treated with 2000 ppm of the inhibiting agent being tested (or, for the controls, the same untreated Mash). The treatment of the Mash was effected by blending it with a small amount of an acetone solution of the agent to be tested. The mice were then sacrificed on Day 10, except for the mouse treated with the agent designated as 6j; the latter was sacrificed on Day 7. The tissues were refrigerated at 4°C overnight, then blotted dry, and the fresh weight of each tissue sample was recorded. The tissues were then homogenized in 5 ml, or 10 ml in the cases of intestines and liver, of acetone-0.1 N $NH_4OH$ (9:1, v/v). The homogenates were then centrifuged at 1500 g and the supernatants were analyzed on a SPEX FA112 spectrofluorometer; excitation 400 nm, emission 630 nm wavelengths, the optima for Proto IX. Proto IX accumulation was determined as CPS (fluorescent counts per second emitted) per gram of tissue. The results are tabulated below (results are averages for two mice for each treatment, except for treatments with agents 6f, 6h, 6i and 6j in each of which only one mouse was treated):

Proto IX Mean Cps per gram x $10^{3}$*

| Agent | Tumor | Liver | Kidneys | Adrenal | Intestine | Weight of Tumor as excised (mg) |
|---|---|---|---|---|---|---|
| 6a | 1203 | 4291 | 10817 | 6572 | 5870** | 143 |
| 6b | 1520 | 1261 | 2441 | 2529 | 5577** | 90 |
| 6c | 14336 | 2739 | 16186 | 13790 | 9937** | 157 |
| 6d | 2006 | 1581 | 12865 | 443 | 5565** | 294 |
| 6e | 837 | 876 | 915 | 1331 | 2069** | 165 |
| 6f | 449 | 1170 | 661 | 5611 | 1157 | 69 |
| 6g | 166 | 803 | 606 | 1984 | 466 | 250 |
| 6h | 298 | 914 | 585 | 2259 | 584 | 192 |
| 6i | 932 | 1510 | 4014 | 36940 | 3454 | 20 |
| 6j | 1251 | 538 | 315 | 491 | 572 | 11 |
| Control | 239 | 536 | 494 | 1504 | 233<br>424** | 200 |

* Numbers in the table equal raw data x $10^{-3}$.

** Measurements were made on samples diluted with 3 parts basic acetone to one part sample.

These figures correspond to the following concentrations of Proto IX in the respective tissues, expressed as ug of Proto IX per gram of tissue:

| Agent | Tumor | Liver | Kidneys | Adrenal | Intestine |
|---|---|---|---|---|---|
| 6a | 11 | 39 | 99 | 60 | 54 |
| 6b | 14 | 12 | 22 | 23 | 51 |
| 6c | 131 | 25 | 148 | 126 | 91 |
| 6d | 18 | 14 | 117 | 4 | 51 |
| 6e | 8 | 8 | 8 | 12 | 19 |
| 6f | 4 | 11 | 6 | 51 | 11 |
| 6g | 2 | 7 | 6 | 18 | 4 |
| 6h | 3 | 8 | 5 | 21 | 5 |
| 6i | 9 | 14 | 37 | 337 | 32 |
| 6j | 11 | 5 | 3 | 4 | 5 |
| Control | 2 | 5 | 5 | 14 | 2<br>4 |

The article by Dougherty cited above gives a figure of 3.6gμg/g for the concentration of the porphyrin in the same type (SMT-F) of tumor after injection of 10mg/kg of hematoporphyrin derivative into the same type (DBA/2 Ha) of mice. Other technical literature (Moan et al P&P 46-5, pages 713-721; note fig 2 on page 716) indicates that a concentration of about 12μg/g of the porphyrin is attained in tumors ($C_3H$/Tif mammary carcinoma) in DBA/H2 mice after intraperitoneal injection of 25mg/kg of Photofrin II, the widely used sensitizer for

photodynamic treatment and detection of cancer.

The total amounts of agent-containing feed consumed by the mice were as follows: 6a, 22 and 35g; 6b, 37 and 35g; 6c, 25 and 22g; 6d, 41 and 32g; 6e, 40 and 44g; 6f, 27g; 6g, 33 and 40g; 6h, 36g; 6i, 10g; 6j, 20g.

In preparation for the experiments described in this Example 4, the following routine steps were carried out, using (unless otherwise noted) the agent which is designated 6c in that Example, with non-tumor-bearing animals:

a. The $LD_{50}$ of the agent was determined to be over 2600 mg/kg (i.e. mg of the agent per kg of body weight) for rats (determined during a 14-day period following a single oral dose comprising corn oil containing 15% of the agent) and over 700 mg/kg for mice (determined during a 14-day period following a single oral dose comprising corn oil and 5% of the agent).

b. In tests of vehicles for injection intra-peritoneally, without the agent, it was determined that the mice could tolerate injection of a 0.5 ml dose of a mixture of equal parts of DMSO (dimethylsulfoxide) and water.

c. In tests of the agent injected intra-peritoneally in a mixture of 60% corn oil and 40% DMSO it was determined that the mice could tolerate a dose of 100 mg/kg.

d. The following trials were carried out with mice:

i. daily oral gavage of 50 mg/kg (using a 1% solution of the agent in acetone) for eight days;

ii. daily I.P. injection of 50 mg/kg (using a 1% dispersion of the agent in mineral oil, made by dissolving the agent in a drop of DMSO and mixing the resulting solution with the mineral oil) for eight days;

iii. daily topical application to the skin of 50 mg/kg (using a 1% solution in DMSO) for eight days;

iv. feeding for eight days of a standard feed into which there had been incorporated 2000 ppm of the agent, based on the weight of the feed;

v. daily I.V. injection of 50 mg/kg (using a 2% solution in DMSO) for four days.

The mice used in these trials were then sacrificed and their tissues examined for Proto IX accumulation.

In another test, a male Fisher 344 rat was fed for 27 days with feed containing 5000 ppm of agent 6c and then sacrificed. The tissues of this rat showed increased levels of Proto IX as compared to a control, with marked increases in the levels in the kidney, intestine, stomach and brain and only a small increase in the level in muscle tissue.

In the foregoing tests of rats and mice the animals were kept on a standard 12 hr. dark-12hr. light cycle.

## EXAMPLE 5

In a series of experiments along the lines of Examples 1 and 2, cultures of HeLa cells were incubated in the presence of 100uM of various treating agents, listed below. The percentage given, next to each treating agent, indicates the increase in the amount of Proto IX produced in the presence of that treating agent relative to the amount produced by the control in the same experiment.

Agents used in Examples 4: 6a, 337%; 6b, 366%; 6c, 297%; 6d, 1200%; 6e, 1210%; 6f, 280%; 6g, 326%; 6h, 431%; 6i, 544%; 6j, 469%. Other agents:

7a.

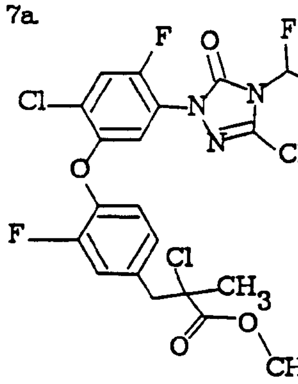

136%

7b.

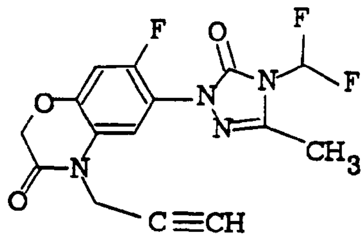

229%

7c.

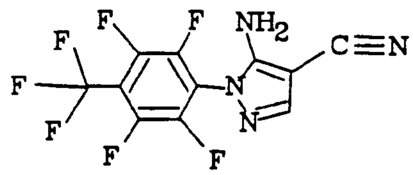

20%

7d.

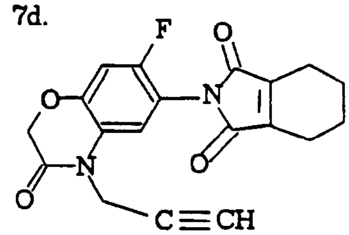

115%

7e.

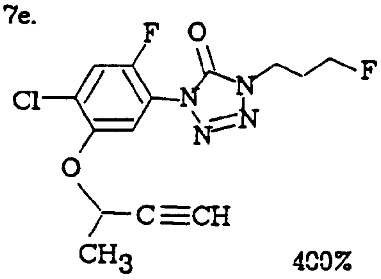

400%

7f.

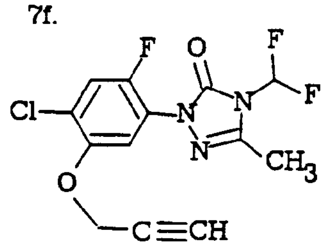

122%

7g.

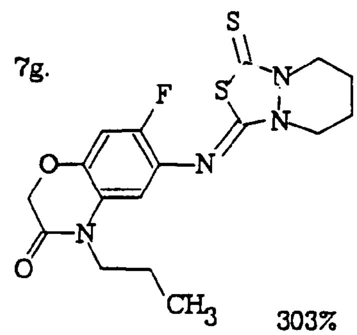

303%

7h.

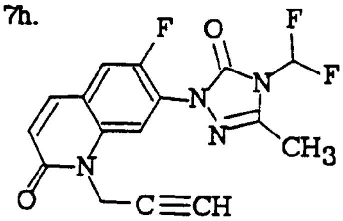

97%

EXAMPLE 6

In this Example the enzyme-inhibiting agent 6c of Example 4 was fed to rats, tumors were implanted in the rats and the tumor-bearing zone was exposed to light, causing regression of the tumors.

Specifically, three Sprague-Dawley rats of average weight 120 g were placed on a diet of feed containing 2000 ppm of the agent for a period of 6 days. On day 3, chondrosarcoma was implanted into the right hind limb of each rat by injecting 0.3 ml of a suspension of the tumor cells (one million tumor cells). On day 6 the right hind limb of each rat was shaven and depilated, the size of each tumor was measured, and the tumor area and surrounding skin were then exposed to non-thermal doses of 630 nm light for a total of 270 J/cm$^2$. On the next day, two of the rats were found to be apparently free of their tumors (that is, no palpable tumor mass), and the third rat showed almost complete regression of its tumor. The surrounding skin and muscle tissue of the rats were slightly blanched and showed some swelling, significantly less than is encountered with Photofrin II-mediated photodynamic therapy.

## Appendix A
## Percent Efflux Test

The Percent Efflux test employs cotyledons harvested from etiolated cucumber seedlings. In an initial step a mass of cotyledons is treated in the dark with an aqueous buffered solution containing a radiolabelled sugar. The amount of the sugar taken up by the cotyledons is then measured (by counting, as described below). The mass of cotyledons is then divided into two portions; one portion of the cotyledons is treated in the dark with an aqueous buffered solution containing the compound to be tested, while the other portion is

treated in the same way, in the dark, with an otherwise identical solution without the test compound, as a control. The cotyledons, in contact with the aqueous solutions, are then exposed to light for 16 hours, and then separated from the aqueous solutions; the latter are then measured (by counting) to determine their contents of radiolabelled material. The results are expressed as Percent Efflux which is calculated as follows, where S is the count of radiolabelled material (per cotyledon) taken up by the cotyledons in their initial treatment, $S_T$ is the count of radiolabelled material (per cotyledon) in the aqueous solution containing the compound to be tested, after the exposure to light and $S_C$ is the count of radiolabelled material (per cotyledon) in the aqueous solution of the control, after exposure to light:

$$\text{Percent Efflux} = \frac{S_T \text{ minus } S_C}{S}$$

More specifically, the following materials and conditions are employed in the Percent Efflux test.

Plant material: Cucumber seed (*Cucumis sativus* L. cultivar 'Wisconsin SMR 18') was germinated and grown in vermiculite irrigated with a commercial (9-45-15) fertilizer. Seedlings were grown at 25°C and 80-90% RH in a dark incubation chamber. Cotyledons were harvested from the etiolated seedlings five days after planting and were rinsed in 1.0 mM $CaCl_2$, all under green light.

Buffered solution: 1 mM KCl, 1mM $CaCl_2$ and 2.0mM potassium phosphate, adjusted to pH 6.5 (as with NaOH).

Radiolabelled sugar: 3-0-methyl-3-[U-14C]glucose of specific activity 10.9 GBq/mmol (Amersham Corp., Arlington Heights IL).

Initial treatment: Washed cotyledons (180 to 230) are added to a 250 mL widemouth foam-stoppered

Erlenmeyer flask containing 50 mL of the buffered solution, and the radiolabelled sugar is added in an amount to give a concentration of 600 nM thereof in the solution. The flask is shaken for 24 hours at 125 rpm on a gyratory shaker in the dark. The cotyledons are then recovered on a nylon mesh and rinsed three times in 20 mL volumes of 1mM $CaCl_2$. The uptake of the radiolabelled sugar is measured by digesting three samples of five cotyledons each in NCS tissue solubilizer (Amersham Corp.) and counting the resulting macerate in a liquid scintillation spectrometer. (Results from these digestions were found to be equivalent to the same determination made by combusting sampled cotyledons in an autooxidizer.)

Treatment with compound to be tested (and control treatment):

Five of the cotyledons are floated, abaxial side up, on 3 mL of the buffered solution in a 35 mm diameter covered plastic petri dish. The test compound is then added (as a solution thereof in acetone) in such amount as to give a predetermined concentration (discussed below) of the test compound in the buffered solution; the acetone concentration in the buffered solution is then 0.1% (v/v) in the control as well as in the solution containing the test compound. The floating cotyledons are then swirled by shaking the dishes at 90 rpm on the surface of a gyratory shaker for 16 hours.

Exposure to light: The dishes containing the cotyledons floating on the solutions are exposed for 16 hours to illumination provided by four GE F20T12-CW fluorescent lamps at a measured intensity of 150$\mu$E $m^{-2}$ $sec^{-1}$ in the photosynthetically active region of the spectrum (PAR) (measured at the surface of the cotyledons) while the dishes are being shaken.

Measurement of amount of radiolabelled material in

the liquid: All the liquid is separated from the cotyledons and then counted in a liquid scintillation spectrometer.

Darkness: All treatments prior to the illumination step are carried out in the dark or under green fluorescent light (i.e. light filtered through a green plastic cut-off filter which passes light of 450-600 nm).

## Appendix B

### Culture Media

#### Medium M

| Salts | Molarity | Stock | mL Stock/L |
|---|---|---|---|
| Na Citrate·$6H_2O$ | $1.7 \times 10^{-3}M$ | 10% | 5 |
| Trace Metals | as below | as below | 10 |
| $FeCl_3 \cdot 6H_2O$ | $0.37 \times 10^{-3}M$ | 1% | 1 |
| $CaCl_2 \cdot 2H_2O$ | $0.36 \times 10^{-3}M$ | 5.3% | 1 |
| $MgSO_4 \cdot 7H_2O$ | $1.2 \times 10^{-3}M$ | 10% | 3 |
| $NH_4NO_3$ | $3.7 \times 10^{-3}M$ | 10% | 3 |
| $KH_2PO_4$ | $2.2 \times 10^{-3}M$ | 10% | 3 |
| $K_2HPO_4$ | $1.7 \times 10^{-3}M$ | 10% | 3 |

#### Stock of Trace Metals Mixture

| | |
|---|---|
| $H_3BO_3$ | 100 mg/L |
| $ZnSO_4 \cdot 7H_2O$ | 100 mg/L |
| $MnSO_4 \cdot 4H_2O$ | 40 mg/L |
| $COCl_2 \cdot 6H_2O$ | 20 mg/1L |
| $NaMoO_4 \cdot 2H_2O$ | 20 mg/L |
| $CuSO_4$ | 4 mg/L |

Culture Medium A is prepared by adding 10 mL/L of an aqueous, 10% sodium acetate solution ($7.5 \times 10^{-3}M$) to Medium M.

The components are added to distilled water in the order listed above and are autoclaved at 100KPa (15 psi) for 15 minutes.

Stock Cultures Grown in the Presence of Light

Stock Cultures of y-1 cells are maintained axenically in liquid culture on Medium A or M, preferably on Medium M, in a 14/10 hour light/dark cycle at 25°C in Erlenmeyer flasks which are stoppered with polyurethane plugs. The stock cultures are incubated without supplemental aeration on rotary shakers at 125 rpm. The light intensity at the culture level is 120 $\mu E\ m^{-2}\ sec^{-1}$ (PAR). Under these conditions the cultures are in a semi-synchronous mode of growth until they reach a stationary phase of 2-4 x $10^6$ cells/mL.

Cultures Grown Without Light (Dark-Grown Culture)

Cells from the stationary phase of stock cultures grown in the presence of light (7.5 mL) are transferred to 750 mL of Medium A in an Erlenmeyer flask. The cells are incubated in the dark, with aeration through a submersed aeration tube, at 25°C for three to four days. During this period this culture of y-1 cells will undergo seven to eight cell divisions, losing all visible chlorophyll, and the concentration should be 2-3 x $10^6$ cells/mL.

Preparation of Cells for Use in Assay

Cells are harvested from a freshly prepared dark-grown culture (750 mL) by low speed centrifugation, i.e., about 2000 rpm, for approximately five minutes at about 20°C. The cells are gently resuspended in 50 mL of Medium A. A sample of this suspension (approximately 0.25 mL) should be examined for motility and, after fixation with an aqueous, 1% gluteraldehyde solution, a cell count should be made to determine the number of cells/mL. A normal cell count is about 5 x $10^7$ cells/mL. Aliquots of 1 mL ($10^7$ cells/mL) are placed into the testing vessels (e.g., Falcon® 24-well tissue

culture plates). At this point the cells are very motile and are yellow in color.

The test compound is dissolved in a solvent (acetone, ethanol, dimethylsulfoxide, or water, preferably acetone) to provide a concentration which is 1000 times the final concentration. One μL of this test solution is added to each of two wells with a 5 or 10 μL microsyringe.

Four control wells per tissue culture plate are prepared in the manner described above without the test compound present. The tissue culture plates are covered with a transparent plastic cover and are placed in a growth chamber with a light intensity of 70-90 $\mu E\ m^{-2}\ sec^{-1}$ for 13-16 hours at 25°C. If the contents of the test wells appear yellow, they are extracted as described in the Evaluation of Results section below. If the contents of the test wells appear transparent or are a pale-green color, they are placed on a rotary shaker at approximately 125 rpm and are irradiated for two hours with a light intensity of about 600 $\mu E\ m^{-2}\ sec^{-1}$ before being extracted.

Evaluation of Results

An aqueous, 10% sodium dodecyl sulfate solution (250 μL) and methanol (1 mL) is added to each test well and is mixed thoroughly. The mixtures are allowed to stand in the dark for three to four hours. The tissue culture plates are centrifuged at room temperature at low speed (ca. 2000 rpm) for five minutes. The supernatants are removed and analyzed in a Beckman Model 35 spectrophotometer between 350 nm and 500 nm for the presence of protophorphrin IX, at 668 nm which is the absorption peak of chlorophyll in this solvent system, and at 720 nm to use as a turbidity background reading.

Analysis of Data

For each well a greenness value is obtained by subtracting the 720 nm reading from the 668 nm reading. These values are averaged for each concentration of the compound to be tested and for the control. The percent inhibition is

$$100 \times \left(1 - \frac{\text{Average greenness value for the particular concentration}}{\text{Average greenness value for the control}}\right)$$

## Claims

1. A pharmaceutical composition for use in the photodynamic treatment or detection of tumors, said composition being characterized by a compound which is a specific inhibitor of the enzymatic conversion of protoporphyrinogen to protoporphyrin IX by protoporphyrinogen oxidase, said compound having an $I_{50}$ for protoporphyrinogen oxidase of less than 1 μM, said composition also comprising a pharmaceutically acceptable vehicle.

2. A pharmaceutical composition for use in photodynamic treatment or detection of tumors, said composition being characterized by a compound which is a specific inhibitor of the enzymatic conversion of protoporphyrinogen to protoporphyrin IX by protoporphyrinogen oxidase, said compound not being a tetrapyrrole and having a redox potential more negative than -500mV, said composition also comprising a pharmaceutically acceptable vehicle.

3. An inhibitor of the enzymatic conversion of protoporphyrinogen to protoporphyrin IX by protoporphyrinogen oxidase, said inhibitor being a specific inhibitor having an $I_{50}$ for protoporphyrinogen oxidase of less than 1 μM, for therapeutic use.

4. An inhibitor of the enzymatic conversion of protoporphyrinogen to protoporphyrin IX by protoporphyrinogen oxidase, said inhibitor being a specific inhibitor and having an $I_{50}$ for protoporphyrinogen oxidase of less than 1 μM, for use in the photodynamic treatment of tumors.

5. An inhibitor of the enzymatic conversion of protoporphyrinogen to protoporphyrin IX by protoporphyrinogen oxidase, said inhibitor being a specific inhibitor and not being a tetrapyrrole and having a redox potential more negative than -500 mV, for therapeutic use.

6. An inhibitor of the enzymatic conversion of protoporphyrinogen to protoporphyrin IX by protoporphyrinogen oxidase, said inhibitor being a specific inhibitor and not being a tetrapyrrole and having a redox potential more negative than -500 mV, for use in the photodynamic treatment of tumors.

7. Inhibitor as in any of claims 3 to 6 which is 1-(4-chloro-2-fluoro-5-propargyloxyphenyl)-3-methyl-4-difluoromethyl-$\Delta^2$-1,2,4-triazolin-5-one.

8. Inhibitor as in claim 4 or 6 which is an aryl triazolinone, an aryl tetrazolinone, an aryl triazinedione, an aryl oxadiazole, an aryl tetrahydroindazole, an aryl tetrahydrophthalimide, an aryl hydantoin, an aryl urazole, an aryl hexahydropyridazine, an aryl heterocyclic urethane, a phenoxyphenyl compound, an aryl pyrazole, or a compound of the formula

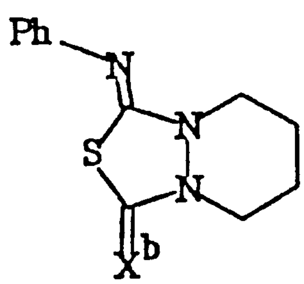

or

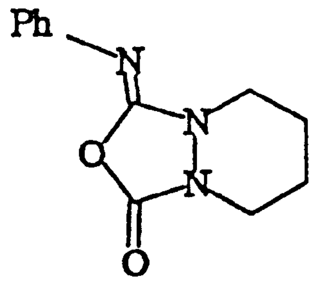

where $x^b$ is O or S and "Ph" is a substituted phenyl.

9. Inhibitor as in claim 8 in which the aryl group has a substituent which is an acidic sulfonamide group or a water-soluble salt thereof.

10. Inhibitor as in claim 9 which is an aryl triazolinone.

11. Inhibitor as in claim 10 having the formula

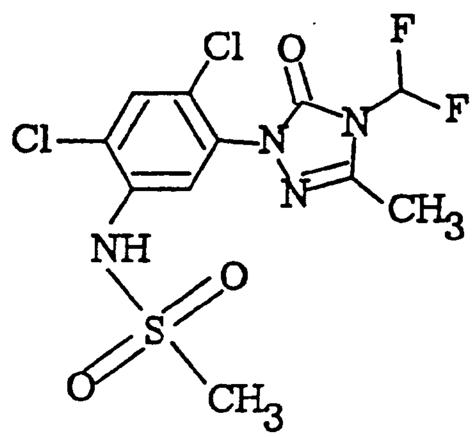

12. Inhibitor as in claim 8 in an orally acceptable vehicle.

13. Inhibitor as in claim 8 in sterile condition.

14. Inhibitor as in claim 8 for use, by oral administration, in the photodynamic treatment of tumors, said inhibitor being a water-soluble salt or an a acid compound which forms a water-soluble sodium or potassium salt.

15. Use of a compound which is a specific inhibitor of the enzymatic conversion of protoporphyrinogen to protoporphyrin IX by protoporphyrinogen oxidase, said compound having an $I_{50}$ for protoporphyrinogen oxidase of less than 1 $\mu$M, for the manufacture of a pharmaceutical composition for use in photodynamic treatment or detection of tumors.

16. Use of a compound which is a specific inhibitor of the enzymatic conversion of protoporphyrinogen to protoporphyrin IX by protoporphyrinogen oxidase, said compound not being a tetrapyrrole and having a redox potential mote negative than -500 mV, for the manufacture of apharmaceutical composition for use in photodynamic treatment or detection of tumors.

17. Use as in claim 15 or 16, wherein said compound is 1-(4-chloro-2-fluoro-5-propargyloxyphenyl)-3-methyl-4-difluoromethyl-$\Delta^2$-1,2,4-triazolin-5-one.

18. Use as in claim 15 or 16, wherein said compound is an aryl triazolinone, an aryl tetrazolinone, an aryl triazinedione, an aryl oxadiazole, an aryl tetrahydroindazole, an aryl tetrahydrophthalimide, an aryl hydantoin, an aryl urazole, an aryl hexahydropyridazine, an aryl heterocyclic urethane, a phenoxyphenyl compound, an aryl pyrazole, or a compound of the formula

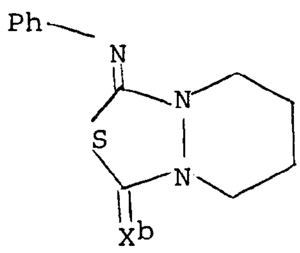

or

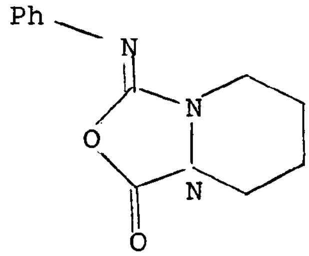

where $x^b$ is O or S and "Ph" is a substituted phenyl.

19. Use as in claim 18 in which the aryl group has a substituent which is an acidic sulfonamide group or a water-soluble salt thereof.

20. Use as in claim 19, where said compound is an aryl triazolinone.

21. Use as in claim 20, wherein said compound has the formula

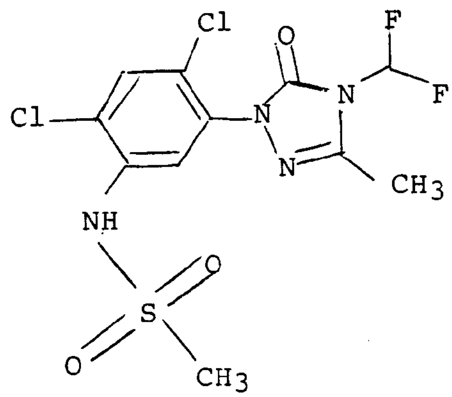

## Patentansprüche

1. Pharmazeutische Zusammensetzung zur Verwendung in der photodynamischen Behandlung oder Erkennung von Tumoren, gekennzeichnet durch eine Verbindung, bei welcher es sich um einen spezifischen Inhibitor der Enzymkonversion von Protoporphyrinogen in Protoporphyrin IX durch Protoporphyrinogenoxidase handelt, wobei in dieser Verbindung $I_{50}$ für die Protoprophyrinogenoxidase einen Wert von unter 1 µm aufweist und die Zusammensetzung des weiteren eine pharmazeutisch verträgliche Trägersubstanz enthält.

2. Pharmazeutische Zusammensetzung zur Verwendung in der photodynamischen Behandlung oder Erkennung von Tumoren, gekennzeichnet durch eine Verbindung, bei welcher es sich um einen spezifischen Inhibitor der Enzymkonversion von Protoporphyrinogen in Protopophyrin IX durch Protoporphyrinogenoxidase handelt, welche Verbindung kein Tetrapyrrol ist und ein Redoxpotential von unter -500mV aufweist, wobei diese Zusammensetzung des weiteren eine pharmazeutisch verträgliche Trägersubstanz enthält.

3. Inhibitor der Enzymkonversion von Protoporphyrinogen in Protoporphyrin IX durch Protoporphyrinogenoxidase zum therapeutischen Einsatz, bei welchem es sich um einen spezifischen Inhibitor handelt, in welchem $I_{50}$ für die Protoporphyrinogenoxidase einen Wert von unter 1µm aufweist.

4. Inhibitor der Enzymkonversion von Protoporphyrinogen in Protoporphyrin IX durch Protoporphyrinogenoxidase zum Einsatz bei der photodynamischen Behandlung von Tumoren, bei welchem es sich um einen spezifischen Inhibitor handelt, in welchem $I_{50}$ für die Protoporphyrinogenoxidase einen Wert von unter 1 µm aufweist.

5. Inhibitor der Enzymkonversion von Protoporphyrinogen in Protoporphyrin IX durch Protoprophyrinogenoxidase

zur therapeutischen Verwendung, bei welchem es sich um einen spezifischen Inhibitor und nicht um ein Tetrapyrrol handelt und ein Redoxpotential von unter -500mV aufweist.

6. Inhibitor der Enzymkonversion von Protoporphyrinogen in Protoporphyrin IX durch Protoporphyrinogenoxidase zum Einsatz bei der photodynamischen Behandlung von Tumoren, bei welchem es sich um einen spezifischen Inhibitor und nicht um ein Tetrapyrrol handelt und welcher ein Redoxpotential von unter -500 mV aufweist.

7. Inhibitor nach einem der Ansprüche 3 bis 6, bei welchem es sich um 1-(4-Chlor-2-fluor-5-propargyloxyphenyl)-3-methyl-4-difluormethyl- $\Delta^2$-1,2,4-triazolin-5-eins) handelt.

8. Inhibitor nach Anspruch 4 oder 6, bei welchem es sich um ein Aryltriazolin, ein Aryltetrazolinon, ein Aryltriazindinon, ein Aryloxadiazol, ein Aryltetrahydroindazol, ein Aryltetrahydrophthalimid, ein Arylhydantoin, ein Arylurazol, ein Arylhexahydropyridazin, ein Arylheterourethan, eine Phenoxyphenylverbindung, ein Arylpyrazol oder eine Verbindung folgender Formel handelt:

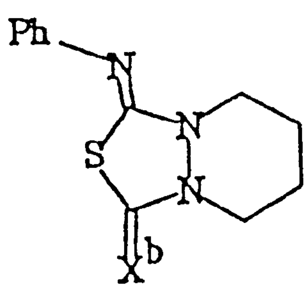

or

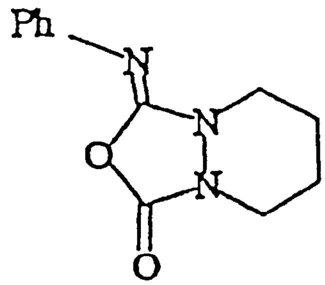

in welcher $x^b$ = O oder S und "Ph" ein substituiertes Phenyl ist.

9. Inhibitor nach Anspruch 8, dadurch gekennzeichnet, dass die Arylgruppe einen Substituenten aufweist, bei welchem es sich um eine Säuresulfonamidgruppe oder ein wasserlösliches Salz derselben handelt.

10. Inhibitor nach Anspruch 9, bei welchem es sich um ein Aryltriazolinon handelt.

11. Inhibitor nach Anspruch 10 folgender Formel

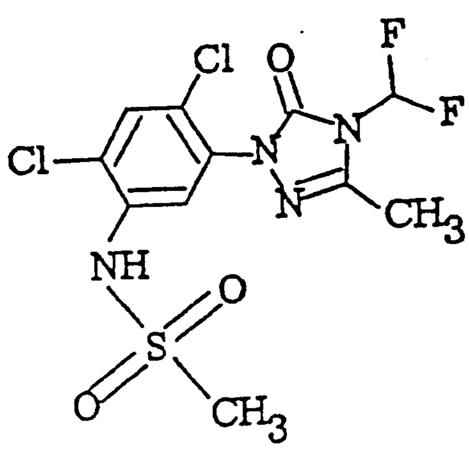

12. Inhibitor nach Anspruch 8, bei welchem es sich um eine oral verträgliche Trägersubstanz handelt.

13. Inhibitor nach Anspruch 8 in sterilem Zustand.

14. Inhibitor nach Anspruch 8 zur oral zu verabreichenden Verwendung bei der photodynamischen Behandlung von Tumoren, dadurch gekennzeichnet, dass der Inhibitor ein wasserlösliches Salz oder eine Säureverbindung ist, welche ein wasserlösliches Natrium- oder Kaliumsalz bildet.

**15.** Verwendung einer Verbindung, bei welcher es sich um einen spezifischen Inhibitor der Enzymkonversion von Protoporphyrinogen in Protoporphyrin IX durch Protoporphyrinogenoxidase handelt, in welcher $I_{50}$ für die Protoporphyrinogenoxidase einen Wert von unter 1 μm aufweist, zur Herstellung einer pharmazeutischen Zusammensetzung zum Einsatz bei der photodynamischen Behandlung oder Erkennung von Tumoren.

**16.** Verwendung einer Verbindung, bei welcher es sich um einen spezifischen Inhibitor der Enzymkonversion von Protoporphyrinogen in Protoporphyrin IX durch Protoporphyrinogenoxidase handelt, wobei die Verbindung kein Tetrapyrrol ist und ein Redoxpotential von unter -500mV aufweist, zur Herstellung einer pharmazeutischen Zusammensetzung zum Einsatz bei der photodynamischen Behandlung oder Erkennung von Tumoren.

**17.** Verwendung nach Ansprüchen 15 oder 16, dadurch gekennzeichnet, dass die Verbindung ein 1-(4-Chlor-2-fluor-5-propargyloxyphenyl)-3-methyl-4-difluormethyl- $\Delta^2$-1,2,4-triazonlin-5-eins ist.

**18.** Verwendung nach Ansprüchen 15 oder 16, dadurch gekennzeichnet, dass die Verbindung ein Aryltriazolinon, ein Aryltetrazolinon, ein Aryltriazinedion, ein Aryloxadiazol, ein Aryltetrahydroindazol, ein Aryltetrahydrophthalimid, ein Arylhydantoin, ein Arylurazol, ein Arylhexahydropyridazin, ein Arylherourethan, eine Phenoxyphenyl-Verbindung, ein Arylpyrazol oder eine Verbindung folgender Formel ist:

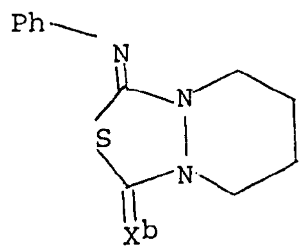

or

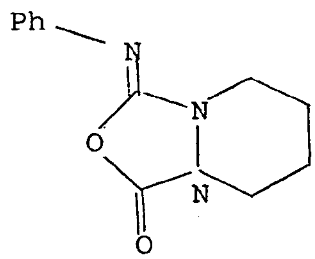

in welcher $x^b$ = O oder S und "Ph" ein substituiertes Phenyl ist.

**19.** Verwendung nach Anspruch 18, dadurch gekennzeichnet, dass die Arylgruppe einen Substituenten aufweist, bei welchem es sich um eine Säuresulfonamidgruppe oder ein wasserlösliches Salz desselben handelt.

**20.** Verwendung nach Anspruch 19, dadurch gekennzeichnet, dass die Verbindung ein Aryltriazolin ist.

**21.** Verwendung nach Anspruch 20, dadurch gekennzeichnet, dass die Verbindung folgende Formel aufweist:

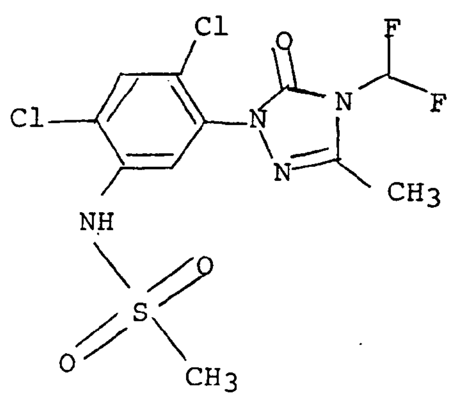

## Revendications

1. Composition pharmaceutique pour utilisation dans le traitement ou la détection photodynamique de tumeurs, ladite composition étant caractérisée par un composé qui est un inhibiteur spécifique de la conversion enzymatique du protoporphyrinogène en protoporphyrine IX par la protoporphyrinogène oxydase, ledit composé ayant une $CI_{50}$ pour la protoporphyrinogène oxydase inférieure à 1 μM, ladite composition comprenant également un véhicule pharmaceutiquement acceptable.

2. Composition pharmaceutique pour utilisation dans le traitement ou la détection photodynamique de tumeurs, ladite composition étant caractérisée par un composé qui est un inhibiteur spécifique de la conversion enzymatique du protoporphyrinogène en protoporphyrine IX par la protoporphyrinogène oxydase, ledit composé n'étant pas un tétrapyrrole et ayant un potentiel d'oxydoréduction plus négatif que -500 mV, ladite composition comprenant également un véhicule pharmaceutiquement acceptable.

3. Inhibiteur de la conversion enzymatique du protoporphyrinogène en protoporphyrine IX par la protoporphyrinogène oxydase, ledit inhibiteur étant un inhibiteur spécifique ayant une $CI_{50}$ pour la protoporphyrinogène oxydase inférieure à 1 μM, pour utilisation thérapeutique.

4. Inhibiteur de la conversion enzymatique du protoporphyrinogène en protoporphyrine IX par la protoporphyrinogène oxydase, ledit inhibiteur étant un inhibiteur spécifique et ayant une $CI_{50}$ pour la protoporphyrinogène oxydase inférieure à 1 μM, pour utilisation dans le traitement photodynamique de tumeurs.

5. Inhibiteur de la conversion enzymatique du protoporphyrinogène en protoporphyrine IX par la protoporphyrinogène oxydase, ledit inhibiteur étant un inhibiteur spécifique et n'étant pas un tétrapyrrole et ayant un potentiel d'oxydoréduction plus négatif que -500 mV, pour utilisation thérapeutique.

6. Inhibiteur de la conversion enzymatique du protoporphyrinogène en protoporphyrine IX par la protoporphyrinogène oxydase, ledit inhibiteur étant un inhibiteur spécifique et n'étant pas un tétrapyrrole et ayant un potentiel d'oxydoréduction plus négatif que -500 mV, pour utilisation dans le traitement photodynamique de tumeurs.

7. Inhibiteur selon l'une quelconque des revendications 3 à 6, qui est la 1-(4-chloro-2-fluoro-5-propargyloxyphényl)-3-méthyl-4-difluorométhyl-$\Delta^2$-1,2,4-triazoline-5-one.

8. Inhibiteur selon la revendication 4 ou 6, qui est une aryl-triazolinone, une aryl-tétrazolinone, une aryltriazinedione, un aryl-oxadiazole, un aryl-tétrahydroindazole, un aryl-tétrahydrophtalimide, une arylhydantoïne, un aryl-urazole, une aryl-hexahydropyridazine, un aryl-uréthanne hétérocyclique, un composé phénoxyphényle, un aryl-pyrazole ou un composé de formule

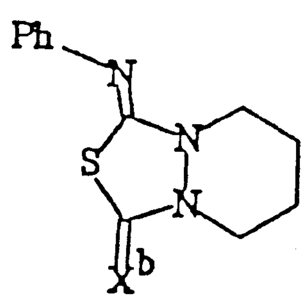

ou

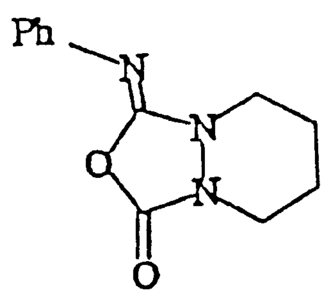

formules dans lesquelles $x^b$ est O ou S et "Ph" est un radical phényle substitué.

9. Inhibiteur selon la revendication 8, dans lequel le groupe aryle comporte un substituant qui est un groupe sulfonamido acide ou un sel hydrosoluble de celui-ci.

10. Inhibiteur selon la revendication 9, qui est une aryl-triazolinone.

11. Inhibiteur selon la revendication 10, de formule

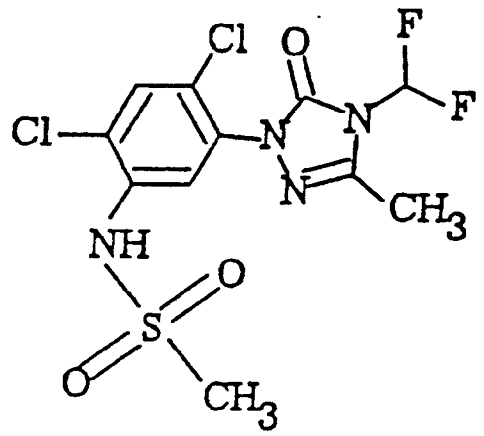

**12.** Inhibiteur selon la revendication 8, dans un véhicule acceptable par voie orale.

**13.** Inhibiteur selon la revendication 8, à l'état stérile.

**14.** Inhibiteur selon la revendication 8, pour utilisation, par administration orale, dans le traitement photodynamique de tumeurs, ledit inhibiteur étant un sel hydrosoluble d'un composé acide qui forme un sel de sodium ou de potassium soluble dans l'eau.

**15.** Utilisation d'un composé qui est un inhibiteur spécifique de la conversion enzymatique du protoporphyrinogène en protoporphyrine IX par la protoporphyrinogène oxydase, ledit composé ayant une $CI_{50}$ pour la protoporphyrinogène oxydase inférieure à 1 μM, pour la préparation d'une composition pharmaceutique pour utilisation dans le traitement ou la détection photodynamique de tumeurs.

**16.** Utilisation d'un composé qui est un inhibiteur spécifique de la conversion enzymatique du protoporphyrinogène en protoporphyrine IX par la protoporphyrinogène oxydase, ledit composé n'étant pas un tétrapyrrole et ayant un potentiel d'oxydoréduction plus négatif que -500 mV, pour la préparation d'une composition pharmaceutique pour utilisation dans le traitement ou la détection photodynamique de tumeurs.

**17.** Utilisation selon la revendication 15 ou 16, dans laquelle ledit composé est la 1-(4-chloro-2-fluoro-5-propargyloxyphényl)-3-méthyl-4-difluorométhyl-$\Delta^2$-1,2,4-triazoline-5-one.

**18.** Utilisation selon la revendication 15 ou 16, dans laquelle ledit composé est une aryl-triazolinone, une aryl-tétrazolinone, une aryl-triazinedione, un aryl-oxadiazole, un aryl-tétrahydroindazole, un aryl-tétrahydrophtalimide, une aryl-hydantoïne, un aryl-urazole, une aryl-hexahydropyridazine, un aryl-uréthanne hétérocyclique, un composé phénoxyphényle, un aryl-pyrazole ou un composé de formule

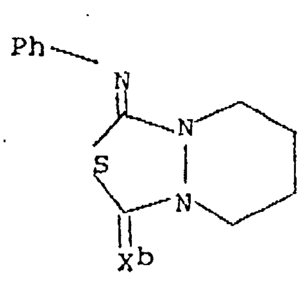

ou

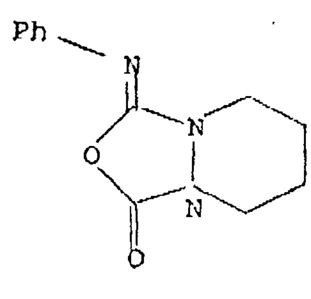

formules dans lesquelles $x^b$ est O ou S et "Ph" est un radical phényle substitué.

**19.** Utilisation selon la revendication 18, dans laquelle le groupe aryle comporte un substituant qui est un groupe sulfonamido acide ou un sel hydrosoluble de celui-ci.

**20.** Utilisation selon la revendication 19, dans laquelle ledit composé est une aryl-triazolinone.

**21.** Utilisation selon la revendication 20, dans laquelle ledit composé est de formule

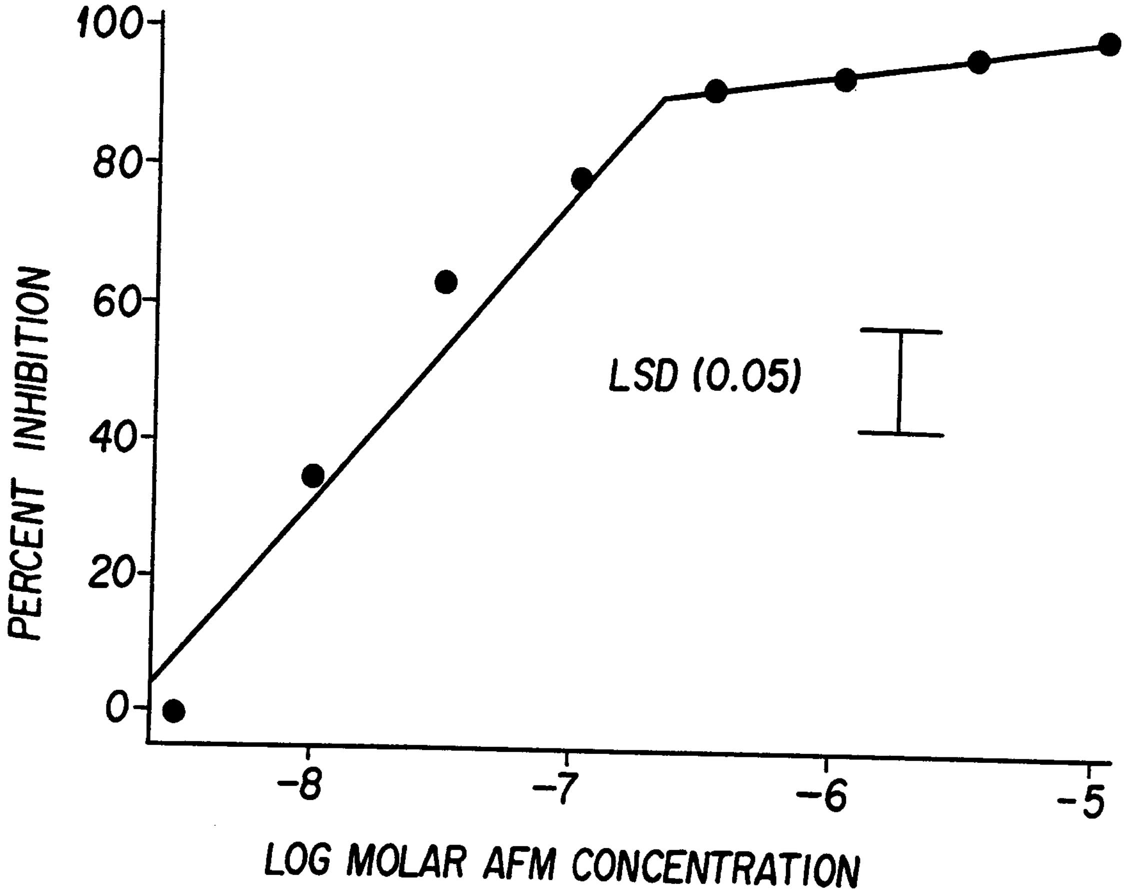
100
80
60
40
20
0
PERCENT INHIBITION
LSD (0.05)
-8
-7
-6
-5
LOG MOLAR AFM CONCENTRATION

FIG. 1